# EUROPÄISCHE PATENTSCHRIFT

(11) **EP 3 482 644 B1**
(45) Veröffentlichungstag und Bekanntmachung des Hinweises auf die Patenterteilung: **09.08.2023**
(21) Anmeldenummer: 17201686.7
(22) Anmeldetag: 14.11.2017
(51) Int. Cl.: A41C 3/00, A61F 7/00, A61F 7/02

(54) **BEHANDLUNGSVORRICHTUNG ZUR WÄRMETHERAPEUTISCHEN BEHANDLUNG BEIDER BRÜSTE EINER PATIENTIN**
TREATMENT DEVICE FOR HEAT TREATMENT OF BOTH BREASTS OF A PATIENT
DISPOSITIF DE TRAITEMENT DESTINÉ AU TRAITEMENT PAR THERMOTHÉRAPIE DES DEUX SEINS D'UNE PATIENTE

(43) Veröffentlichungstag der Anmeldung: 15.05.2019
(73) Patentinhaber: Hilotherm Holding AG, 6317 Oberwil bei Zug (CH)
(72) Erfinder: Stegmann, Christian, 87545 Burgberg im Allgäu (DE)
(74) Vertreter: Wallinger Ricker Schlotter Tostmann

(56) Entgegenhaltungen:
- CN-Y- 2 456 663
- DE-A1-102012 010 355
- DE-U1- 20 014 047
- JP-A- 2011 122 287
- US-A- 3 995 621
- US-A- 5 871 526
- US-A1- 2008 195 185

## Beschreibung

Die vorliegende Erfindung betrifft eine Behandlungsvorrichtung zur Verwendung bei der gleichzeitigen wärmetherapeutischen Behandlung beider Brüste einer Patientin, ein Wärmebehandlungssystem mit einer solchen Behandlungsvorrichtung, eine Fluidversorgungseinrichtung zur fluidleitenden Verbindung mit einer solchen Behandlungsvorrichtung sowie die Verwendung einer solchen Behandlungsvorrichtung und/oder eines solchen Wärmebehandlungssystems zur kosmetischen Behandlung der Haut von Brüsten einer Patientin.

Die thermotherapeutische Behandlung ist ein schon sehr lange bekanntes medizinisches Heilverfahren, bei dem dem Körper Wärme zugeführt wird - man spricht dann von Wärmetherapie - oder dem Körper Wärme entzogen wird - in diesem Fall spricht man von Kältetherapie. Um bei der Wärmetherapie Wärme auf den Körper übertragen zu können, ist die Wärmeübertragung von einer Wärmequelle auf den Körper erforderlich. Bei der Kältetherapie muss hingegen die Wärme dem Körper entzogen werden, d. h. es muss Wärme des Körpers auf eine Wärmesenke übertragen werden.

Die Wärmeübertragung kann in beiden Fällen direkt erfolgen, d. h. indem der Körper selbst in Kontakt mit einem wärmeübertragenden Medium gebracht wird, z. B. in einer Sauna oder in einem kalten Wasserbad. Daneben ist eine indirekte Wärmeübertragung möglich, bei welcher der Körper nicht selbst in Kontakt mit einem wärmeübertragenden Medium kommt. Die vorliegende Erfindung befasst sich mit der letztgenannten Vorgehensweise.

Bei der indirekten Wärmeübertragung wird ein Wärmeübertragungsmedium verwendet, das je nach Anwendung gasförmig oder flüssig, aber auch fest sein kann. Besonders bevorzugt ist als Wärmeübertragungsmedium Wasser, insbesondere destilliertes Wasser. Daneben können aber auch ein Gas oder eine Gasmischung und hier insbesondere Luft oder auch ein Öl oder ein Ölgemisch oder andere geeignete Flüssigkeiten oder Flüssigkeitsgemische zur Anwendung kommen.

Der Prozess der Wärmeübertragung kann diskontinuierlich oder kontinuierlich erfolgen. Eine bekannte diskontinuierliche Wärmeübertragungsanwendung ist der sogenannte Eisbeutel. Dazu wird Wasser zu Eis gefroren und in eine geeignete flexible, meist wasserdicht verschließbare Hülle eingefüllt. Der Eisbeutel wird auf den jeweiligen Körperteil aufgelegt und entzieht dem Körper so lange Wärme, bis das Eis aufgetaut ist und sich die Temperatur des Eisbeutels der Körpertemperatur angenähert hat.

Beim kontinuierlichen Verfahren wird das wärmeübertragende Medium mit einer Temperatur, die je nach Anwendung oberhalb oder unterhalb der Körpertemperatur liegt, einem Wärmeübertragungskörper zugeführt, der in Kontakt mit dem betroffenen Körperteil steht.

Aus der US 2008/0195185 A1 ist eine Vorrichtung für die Wärmetherapie zur Erholung nach Operationen oder Verletzungen der Brustregion mit einer Blase, durch welche eine temperaturgeregeltes Fluid fließt, und einer Hülle, um die Blase zu halten, bekannt. Die Vorrichtung kann an der Vorderseite geöffnet werden, damit sie auf einfache Weise getragen und entfernt werden kann.

Aus der US 3,995,621 ist eine büstenhalterförmige Vorrichtung bekannt, welche mit einem Paar von flüssigkeitsdurchströmten, dem Körper angepassten Kühlplatten ausgestattet ist, welche die Brüste und den oberen Rumpf bedecken. Die Vorrichtung dient der verbesserten Erkennung von Brustkrebs, indem die durch die Vorrichtung gekühlte Brustregion einem termografischen Infrarot-Scan unterzogen wird, auf dem Regionen mit bösartigen Tumoren anhand von deren höherer Temperatur erkannt werden können.

Der vorliegenden Erfindung liegt die Aufgabe zugrunde, die Wärmebehandlung bei der gleichzeitigen wärmetherapeutischen Behandlung beider Brüste einer Patientin zu verbessern.

Diese Aufgabe wird durch eine Wärmebehandlungsvorrichtung zur Verwendung bei der gleichzeitigen wärmetherapeutischen Behandlung beider Brüste einer Patientin gemäß Anspruch 1, einem Wärmebehandlungssystem mit einer solchen Behandlungsvorrichtung gemäß Anspruch 12 sowie durch eine Verwendung einer solchen Behandlungsvorrichtung und/oder eines solchen Wärmebehandlungssystems zur kosmetischen Behandlung der Haut von Brüsten einer Patientin gemäß Anspruch 14 gelöst.

Ein erster Aspekt der Erfindung betrifft eine Behandlungsvorrichtung zur Verwendung bei der gleichzeitigen wärmetherapeutischen Behandlung beider Brüste einer Patientin, insbesondere zur Verwendung bei der Behandlung nach operativen Eingriffen und/oder von Verletzungen und/oder von Entzündungen, mit
- einer ersten und einer zweiten Manschette, welche jeweils wenigstens im Wesentlichen halbkugelförmig ausgebildet ist und mit welcher jeweils eine Brust weitgehend oder vollständig bedeckbar ist, wobei die erste Manschette wenigstens eine erste flexible Materiallage und wenigstens eine zweite flexible Materiallage, welche einen ersten Wärmeübertragungskörper bilden, und die zweite Manschette wenigstens eine dritte flexible Materiallage und wenigstens eine vierte flexible Materiallage, welche einen zweiten Wärmeübertragungskörper bilden, aufweist, wobei jeweils die erste und zweite flexible Materiallage der ersten Manschette und die dritte und vierte flexible Materiallage der zweiten Manschette einen oder mehrere von einem wärmeübertragenden Fluid durchströmbare Strömungsräume begrenzen und durch eine Vielzahl von Verbindungseinrichtungen miteinander verbunden sind;
- einer Befestigungseinrichtung, welche an einem ersten Ende mit der ersten Manschette und an einem zweiten Ende mit der zweiten Manschette verbindbar ist, und zwar im Bereich des Brustbeins der Patientin, wenn die Behandlungsvorrichtung während der Behandlung an der Patientin angeordnet ist;
- einer ersten, flexiblen länglichen Trageinrichtung, welche an einem ersten Ende mit der ersten Manschette und an einem zweiten Ende mit der zweiten Manschette verbindbar ist, sodass durch die erste Trageinrichtung, wenn diese am Hals der Patientin angeordnet ist, die erste und zweite Manschette höhenverstellbar ist;
- einer zweiten, flexiblen länglichen Trageinrichtung, welche an einem ersten Ende mit der ersten Manschette und an einem zweiten Ende mit der zweiten Manschette verbindbar ist, sodass durch die zweite Trageinrichtung, wenn diese den Brustkorb der Patientin umschließt, die erste Manschette an einer ersten Brust und die zweite Manschette an einer zweiten Brust der Patientin fixierbar ist;
- wenigstens einer ersten Zuleitung, um dem ersten und/oder zweiten Wärmeübertragungskörper ein wärmeübertragendes Fluid zuzuführen;
- wenigstens einer zweiten Zuleitung, um ein wärmeübertragendes Fluid von dem ersten und/oder zweiten Wärmeübertragungskörper abzuführen; und
- einer Übertragungseinrichtung, durch welche der erste Wärmeübertragungskörper mit dem zweiten Wärmeübertragungskörper verbindbar ist, sodass das wärmeübertragende Fluid von dem ersten in den zweiten Wärmeübertragungskörper und umgekehrt strömen kann.

Erfindungsgemäß ist dabei die Übertragungseinrichtung (10) im Bereich des Brustbeins (33) der Patientin (30) angeordnet, wenn die Behandlungsvorrichtung (1) während der Behandlung an der Patientin (30) angeordnet ist.

Die Verbindungseinrichtungen, welche die erste und die zweite flexible Materiallage bzw. die dritte und vierte flexible Materiallage der beiden Manschetten verbinden, sind insbesondere in kleinen und regelmäßigen Abständen an der Innenfläche der ersten flexiblen Materiallage und der Innenfläche der zweiten flexiblen Materiallage bzw. an der Innenfläche der dritten flexiblen Materiallage und der Innenfläche der vierten flexiblen Materiallage verteilt und dienen insbesondere dazu, ein Verschließen der Strömungsräume durch ein flächiges Aufeinanderliegen der ersten flexiblen Materiallage und der zweiten flexiblen Materiallage bzw. der dritten flexiblen Materiallage und der vierten flexiblen Materiallage zu verhindern. Die Verbindungseinrichtungen sind vorzugsweise als Schweißpunkte ausgeführt, mit denen die erste flexible Materiallage mit der zweiten flexiblen Materiallage bzw. die dritte flexible Materiallage mit der vierten flexiblen Materiallage verschweißt und dadurch verbunden ist.

In einer bevorzugten Ausgestaltung der Behandlungsvorrichtung weist die Befestigungseinrichtung und/oder die erste Trageinrichtung und/oder die zweite Trageinrichtung jeweils an einem ersten und/oder an einem zweiten Ende wenigstens ein Fixierungselement, insbesondere wenigstens einen Knopf und/oder wenigstens einen Druckknopf und/oder wenigstens ein Lochraster und/oder wenigstens einen Klettverschluss und/oder wenigstens eine Schlaufe, auf, womit die Befestigungseinrichtung und/oder die erste Trageinrichtung und/oder die zweite Trageinrichtung mit der ersten Manschette und/oder der zweiten Manschette verbindbar ist.

Dadurch kann die Behandlungsvorrichtung wiederholt geöffnet bzw. abgelegt und wieder verschlossen werden, wodurch der Zeitraum der Verwendbarkeit der Behandlungsvorrichtung verlängert werden kann. Es ist jedoch auch möglich, dass wenigstens ein Teil der Befestigungseinrichtung und/oder der ersten Trageinrichtung und/oder der zweiten Trageinrichtung mit der ersten und/oder der zweiten Manschette unlösbar, beispielsweise durch Verschweißen, miteinander verbunden ist. Dies erhöht die Langlebigkeit der gesamten Behandlungsvorrichtung.

In einer bevorzugten Variante dieser Ausführung sind die beiden Manschetten auf verschiedene Weise miteinander lösbar verbindbar, so dass dabei verschiedene Größen der Behandlungsvorrichtung entstehen, welche insbesondere verschiedenen Brustgrö-βen einer Patientin entsprechen. Vorzugsweise ist es möglich, dass die Befestigungseinrichtung und/oder die erste Trageinrichtung und/oder die zweite Trageinrichtung mehrere Fixierungselemente, welche insbesondere versetzt zueinander angeordnet sind, aufweist, um eine variable Größenanpassung an die beiden Brüste der Patientin zu ermöglichen. Des Weiteren ist es vorzugsweise möglich, dass die Befestigungseinrichtung und/oder die erste Trageinrichtung und/oder die zweite Trageinrichtung derart ausgebildet ist, dass sie Hilfskräfte auf die Manschetten ausübt, welche zumindest teilweise dazu beitragen, die Manschetten während der Behandlung der Patientin an deren Brüsten zu halten.

In einer weiteren bevorzugten Ausgestaltung der Behandlungsvorrichtung ist die Befestigungseinrichtung und/oder die erste Trageinrichtung und/oder die zweite Trageinrichtung jeweils an einem ersten und/oder an einem zweiten Ende durch das wenigstens eine Fixierungselement längenverstellbar.

Hierdurch ergibt sich eine Vereinfachung bei der Herstellung, der Lagerhaltung und dem Vertrieb der Behandlungsvorrichtung als kommerzielles Produkt, da das Produkt dann nur noch in wenigen oder sogar nur noch in einer Version hergestellt, vorgehalten und ausgeliefert werden muss, ohne dass die Körpermaße der Patientin, insbesondere deren Brustgröße, berücksichtigt werden müssen ("one size fits all").

In einer weiteren bevorzugten Ausgestaltung der Behandlungsvorrichtung weisen die erste und die zweite Manschette jeweils an wenigstens einer Stelle der Außenkante einen Einschnitt auf, welcher in der Aufsicht der ersten oder der zweiten Manschette jeweils wenigstens im Wesentlichen bis zum Kreismittelpunkt der ersten und der zweiten Manschette ausgebildet ist und die erste und zweite Manschette an dem Einschnitt jeweils eine erste und zweite Einschnittkante aufweist.

In einer weiteren bevorzugten Ausgestaltung der Behandlungsvorrichtung weisen die erste und die zweite Manschette jeweils an wenigstens zwei, vorzugsweise drei, weiter vorzugsweise vier, voneinander beabstandeten Stellen der Außenkante einen Einschnitt auf, wobei jeweils die wenigstens zwei Einschnitte der ersten und der zweiten Manschette in der Aufsicht der ersten oder der zweiten Manschette jeweils wenigstens im Wesentlichen bis zum Kreismittelpunkt der ersten und der zweiten Manschette ausgebildet sind und die erste und die zweite Manschette jeweils in wenigstens einen ersten und einen zweiten Teil separieren, wobei die jeweiligen wenigstens ersten und wenigstens zweiten Teile der ersten und zweiten Manschette lediglich im Bereich des Kreismittelpunkts stoffschlüssig miteinander verbunden sind, und die erste und zweite Manschette an den Einschnitten jeweils eine erste und zweite Einschnittkante aufweisen.

Durch diesen Einschnitt bzw. diese Einschnitte kann ermöglicht werden, dass die erste und die zweite Manschette an die jeweilige Brust einer Patientin insofern angepasst werden kann, dass diese die Brüste im Wesentlichen bedecken.

In einer weiteren bevorzugten Ausgestaltung der Behandlungsvorrichtung sind die erste und die zweite Einschnittkante wenigstens jeweils eines Einschnitts der ersten und der zweiten Manschette jeweils mittels wenigstens einer Verstelleinrichtung, insbesondere wenigstens einem Knopf und/oder wenigstens einem Druckknopf und/oder wenigstens einem Lochraster und/oder wenigstens einem Klettverschluss und/oder wenigstens einer Schlaufe, verbindbar, sodass die erste Manschette an die erste Brust und die zweite Manschette an die zweite Brust der Patientin anpassbar ist.

Dadurch kann je nach Form der jeweiligen Brust der Patientin, wie beispielsweise nach einer wenigstens teilweisen Amputation eines Teils einer Brust der Patientin nötig, die erste und die zweite Manschette individuell angepasst werden.

In einer weiteren bevorzugten Ausgestaltung der Behandlungsvorrichtung verlaufen die Strömungsräume wenigstens im Wesentlichen mäanderförmig, insbesondere jeweils von dem wenigstens ersten Teil der ersten oder der zweiten Manschette zu dem wenigstens zweiten Teil der ersten oder der zweiten Manschette.

Durch diesen Verlauf der Strömungsräume wird eine optimierte wärmetherapeutische Behandlung der Brüste der Patientin ermöglicht. Insbesondere kann durch den mäanderförmigen Verlauf von dem ersten zum zweiten Teil der ersten oder zweiten Manschette eine flächendeckende wärmetherapeutische Behandlung gewährleistet werden.

In einer weiteren bevorzugten Ausgestaltung der Behandlungsvorrichtung sind die erste Zuleitung und die zweite Zuleitung im Wesentlichen jeweils an einem Bereich des ersten und des zweiten Wärmeübertragungskörpers angeschlossen, welche sich in Bezug auf den Aufbau der ersten und zweiten Manschette, insbesondere spiegelsymmetrisch, entsprechen.

Dadurch kann eine ausgeglichene Gewichtsverteilung erreicht werden, welche insbesondere bei längeren Anwendungszeiten bzw. Tragzeiten der Behandlungsvorrichtung in einer optimierten Ergonomie resultiert und somit beispielsweise Verspannungen der Patientin aufgrund der Behandlungsvorrichtung und dessen Gewicht verringert, insbesondere verhindert, werden können.

In einer weiteren bevorzugten Ausgestaltung der Behandlungsvorrichtung weisen die Befestigungseinrichtung und/oder die erste Trageinrichtung und/oder die zweite Trageinrichtung ein Kunstfasermaterial auf.

Das Kunstfasermaterial trägt dazu bei, dass die Befestigungseinrichtung und/oder die erste Trageinrichtung und/oder die zweite Trageinrichtung flexibel ausgebildet sind und insbesondere besser an die Körperstatur der Patientin anpassbar sind. Ferner verringert, insbesondere verhindert, das Kunstfasermaterial, dass Rötungen, Schwellungen oder andere hautreizende Phänomene aufgrund des Tragens der Behandlungsvorrichtung am Körper entstehen.

In einer weiteren bevorzugten Ausgestaltung der Behandlungsvorrichtung weisen die Befestigungseinrichtung und/oder die erste Trageinrichtung und/oder die zweite Trageinrichtung einen Kunststoff auf.

Durch den Kunststoff kann eine stabile Ausgestaltung erreicht werden, womit die Behandlungsvorrichtung sicher an der Patientin angeordnet werden kann und die Gefahr eines Verrutschens der Behandlungsvorrichtung während der Anwendung bzw. Behandlung verringert, insbesondere verhindert, wird.

In einer weiteren bevorzugten Ausgestaltung der Behandlungsvorrichtung ist die Vielzahl von Verbindungseinrichtungen zumindest teilweise rasterförmig, vorzugsweise an den Eckpunkten von gedachten Rechtecken, weiter vorzugsweise an den Eckpunkten von gedachten Quadraten, angeordnet.

Dadurch wird erreicht, dass Fluid um die Verbindungseinrichtungen herum in den Wärmeübertragungskörpern strömen kann. Ferner kann somit bei einem Abknicken bzw. Abdrücken eines Strömungsraums des Fluids in der ersten und/oder zweiten Manschette während der Behandlung das Fluid immer noch wenigstens über bzw. in einem alternativen Strömungsraum strömen.

Ein zweiter Aspekt der Erfindung betrifft ein Wärmebehandlungssystem zur Verwendung bei der wärmetherapeutischen Behandlung beider Brüste einer Patientin mit einer erfindungsgemäßen Behandlungsvorrichtung gemäß dem ersten Aspekt und mit einer Fluidversorgungseinrichtung, die mit der Behandlungsvorrichtung fluidleitend verbindbar ist.

In einer bevorzugten Ausführung des Wärmebehandlungssystems gemäß dem zweiten Aspekt ist die Fluidversorgungseinrichtung, mit wenigstens einer ersten Zuleitung der Behandlungsvorrichtung und wenigstens einer zweiten Zuleitung der Behandlungsvorrichtung fluidleitend verbindbar und dazu eingerichtet, ein wärmeübertragendes Fluid auf eine für die Wärmebehandlung geeignete Temperatur, bevorzugt eine Temperatur zwischen +5 °C und +35 °C, weiter bevorzugt zwischen +15 °C und +22 °C, besonders bevorzugt zwischen +17 °C und +19 °C und ganz besonders bevorzugt um ca. +18 °C, zu temperieren, das derart temperierte wärmeübertragende Fluid einem ersten Wärmeübertragungskörper oder einem zweiten Wärmeübertragungskörper der Behandlungsvorrichtung über die erste Zuleitung zuzuführen und das wärmeübertragende Fluid von dem ersten Wärmeübertragungskörper oder dem zweiten Wärmeübertragungskörper über die zweite Zuleitung abzuführen.

Ein dritter Aspekt der Erfindung betrifft die Verwendung einer erfindungsgemäßen Behandlungsvorrichtung gemäß dem ersten Aspekt und/oder eines erfindungsgemäßen Wärmebehandlungssystems gemäß dem zweiten Aspekt zur kosmetischen Behandlung der Haut von Brüsten einer Patientin, insbesondere zur thermischen Beeinflussung der Kapillarzeichnung auf der Haut.

Weitere vorteilhafte Ausgestaltungen der Erfindung werden in der nachfolgenden Beschreibung in Zusammenhang mit den beiliegenden Zeichnungen erläutert. Dabei zeigt:
- **Fig. 1**: eine erfindungsgemäße Behandlungsvorrichtung;
- **Fig. 2**: eine Patientin, welche die erfindungsgemäße Behandlungsvorrichtung trägt; und
- **Fig. 3**: eine detaillierte Ansicht der erfindungsgemäßen ersten und zweiten Manschette in der Aufsicht.

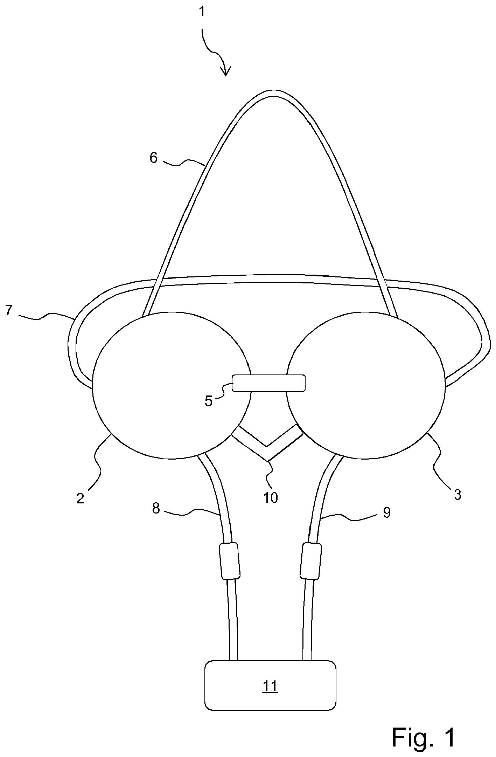

**Fig. 1** zeigt eine erfindungsgemäße Behandlungsvorrichtung 1 zur Verwendung bei der gleichzeitigen wärmetherapeutischen Behandlung beider Brüste 31, 32 einer Patientin 30, insbesondere zur Verwendung bei der Behandlung nach operativen Eingriffen und/oder von Verletzungen und/oder von Entzündungen.

Die erfindungsgemäße Behandlungsvorrichtung 1 weist eine erste Manschette 2 und eine zweite Manschette 3 auf, welche jeweils wenigstens im Wesentlichen halbkugelförmig ausgebildet ist und durch welche jeweils eine erste und zweite Brust 31, 32 einer Patientin 30 weitgehend oder vollständig bedeckt ist.

Ferner weist die Behandlungsvorrichtung 1 eine Befestigungseinrichtung 5 und eine erste und zweite Trageinrichtung 6, 7 auf, mittels welcher die Behandlungsvorrichtung 1 an der Patientin 30 befestigt werden kann. Ausgehend von einer Fluidversorgungseinrichtung 11, welche zur Temperierung eines wärmeübertragenden Fluids vorgesehen ist, wird über eine erste und zweite Zuleitung 8, 9 das wärmeübertragende Fluid in die Behandlungsvorrichtung 1 hinein und auch wieder heraus transportiert. Hierbei gelangt das wärmeübertragene Fluid von der ersten Manschette 2 über eine Übertragungseinrichtung 10 zu der zweiten Manschette 3 oder umgekehrt.

Die erste Manschette 2 weist hierbei wenigstens eine erste flexible Materiallage und eine zweite flexible Materiallage auf, welche zusammen einen ersten Wärmeübertragungskörper bilden. Analog weißt die zweite Manschette 3 wenigstens eine dritte flexible Materiallage und wenigstens eine vierte flexible Materiallage auf, welche einen zweiten Wärmeübertragungskörper bilden. Jeweils die erste und zweite flexible Materiallage der ersten Manschette 2 sowie die dritte und vierte flexible Materiallage der zweiten Manschette 3 begrenzen einen oder mehrere von einem werbetragenden Fluid durchströmte Strömungsräume 12, 12' und sind durch eine Vielzahl von Verbindungseinrichtungen 4, 4', 4", 4‴ miteinander verbunden. Innerhalb dieser beiden Wärmeübertragungskörper strömt das Fluid während der Behandlung der Patientin.

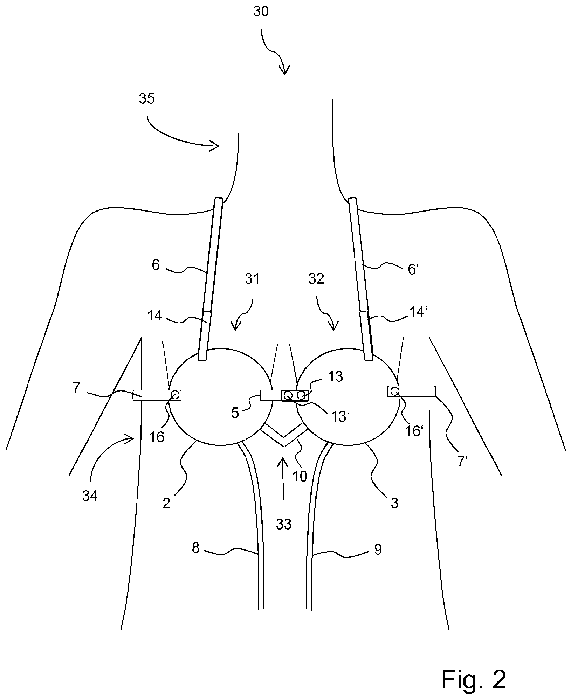

**Fig. 2** zeigt eine Patientin 30, welche die erfindungsgemäße Behandlungsvorrichtung 1 trägt. Die Befestigungseinrichtung 5, welche an einem ersten Ende mit der ersten Manschette 2 und an einem zweiten Ende mit der zweiten Manschette 3 verbindbar ist, erstreckt sich während der Behandlung im Bereich des Brustbeins 33 der Patientin 30. Mittels eines Fixierelements, welches in dieser Darstellung als Lochraster 13, 13' ausgebildet ist, kann eine Weitenverstellung durchgeführt werden, sodass die Behandlungsverrichtung 1 an den Körperbau der Patientin 30 und insbesondere deren Brustgröße angepasst werden kann.

Ferner weist die Behandlungsvorrichtung 1 eine erste, flexible längliche Trageinrichtung 6 auf, welche in einem ersten Ende mit der ersten Manschette 2 und an einem zweiten Ende mit der zweiten Manschette 3 verbindbar ist. Mittels eines weiteren Fixierelements, welches in dieser Darstellung als Klettverschluss 14, 14' an jeweils einem Ende der ersten Trageinrichtung 6, 6' ausgebildet ist, ist es möglich, dass die erste und zweite Manschette 2, 3 höhenverstellbar ist, wenn die erste Trageinrichtung 6 am Hals 35 der Patientin 30 angeordnet ist.

Darüber hinaus ist an der Behandlungsvorrichtung 1 eine zweite, flexible längliche Trageinrichtung 7, 7' angeordnet, welche an einem ersten Ende mit der ersten Manschette 2 und einem zweiten Ende mit der zweiten Manschette 3 verbindbar ist. Wenn somit während der Behandlung der Patientin 30 die Behandlungsvorrichtung 1 mittels der zweiten Trageinrichtung 7, 7' den Brustkorb 34 der Patientin 30 umschließt, ist die erste Manschette 2 an der ersten Brust 31 und die zweite Manschette 3 an der zweiten Brust 32 der Patientin 30 fixierbar. Ferner weist die zweite Trageinrichtung 7, 7' ein weiteres Fixierelement an beiden Manschetten 2, 3 auf, welches in dieser Darstellung als Knopf 16, 16' bzw. Druckknopf ausgebildet ist.

Die Befestigungseinrichtung 5 und/oder die erste Trageinrichtung 6, 6' und/oder die zweite Trageinrichtung 7, 7' können jeweils ein Kunstfasermaterial und/oder auch einen Kunststoff aufweisen. Dies ist insbesondere für den Tragekomfort und somit die Ergonomie der Behandlungsvorrichtung 1 vorteilhaft.

Mittels dieser erfindungsgemäßen Behandlungsvorrichtung 1 bzw. des erfindungsgemäßen Wärmebehandlungssystems kann die Haut an den Brüsten 31, 32 der Patientin 30 kosmetisch behandelt werden, um insbesondere die Kapillarzeichnung auf der Haut mittels einer thermischen Beeinflussung oder auch Hautschwellungen nicht-therapeutisch zu reduzieren. Darüber hinaus kann auch eine thermisch bewirkte Reduzierung der Bildung freier Radikale auf der Hautoberfläche erreicht werden.

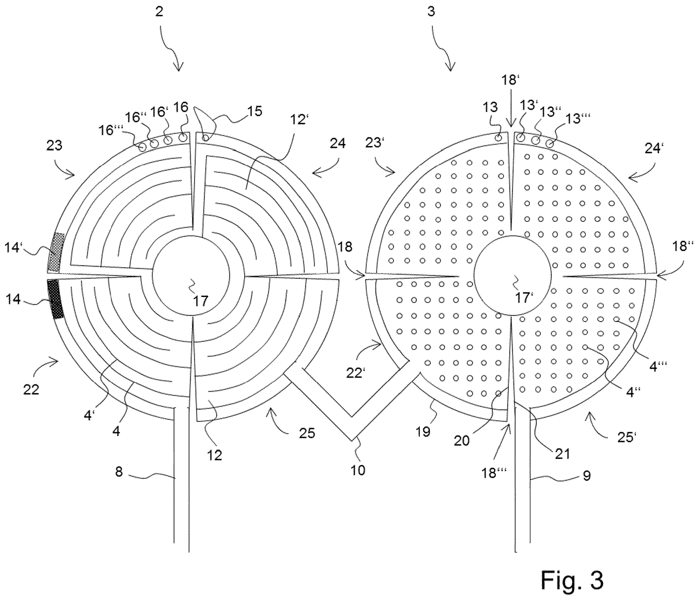

**Fig. 3** zeigt eine detaillierte Ansicht der erfindungsgemäßen ersten und zweiten Manschette 2, 3 in der Aufsicht. Die erste und zweite Manschette 2, 3 ist hierbei jeweils in vier verschiedene Bereiche unterteilt, welche jeweils durch vier beabstandete Einschnitte 18, 18', 18", 18‴ an der Außenkante 19 der jeweiligen Manschette 2, 3 begrenzt sind. Die jeweils vier Teile 22, 22', 23, 23', 24, 24', 25, 25' der beiden Manschetten 2, 3 sind jeweils lediglich im Bereich um den Kreismittelpunkt 17, 17' der jeweiligen Manschette 2, 3 stoffschlüssig miteinander verbunden. Ferner weisen die Einschnitte 18, 18', 18", 18‴ jeweils erste und zweite Einschnittkanten 20, 21 auf.

Mittels einer ersten Zuleitung 8 kann wärmeübertragendes Fluid an die erste Manschette 2 übertragen werden. Hierbei fließt das Fluid in einen ersten Teil 22 der ersten Manschette 2, welcher links unten dargestellt ist. Dadurch wird der Wärmeübertragungskörper der ersten Manschette 2 in verschiedene Strömungsräume 12, 12' mittels einer ersten Vielzahl an Verbindungseinrichtungen 4, 4' unterteilt, sodass das Fluid beim Durchströmen des ersten Teils 22 der ersten Manschette 2 mäanderförmig verläuft. In der zweiten Manschette ist eine zweite Vielzahl von Verbindungseinrichtungen 4", 4‴ zumindest teilweise rasterförmig, vorzugsweise an den Eckpunkten von gedachten Rechtecken, weiter vorzugsweise an den Eckpunkten von gedachten Quadraten, angeordnet. Beide Anordnungen der Verbindungseinrichtungen 4, 4', 4", 4‴ können jeweils einzeln bzw. lediglich oder auch kombiniert verwendet werden.

Ist das Fluid beim Durchströmen des ersten Teils 22 der ersten Manschette 2 im Bereich des Kreismittelpunkts 17 angelangt, fließt es über in einen zweiten Teil 23 der ersten Manschette 2, welcher links oben dargestellt ist. Wiederum verläuft das Fluid mäanderförmig, wobei es anschließend ebenfalls mäanderförmig in einen dritten Teil 24, welcher rechts oben an der ersten Manschette 2 angeordnet ist, und einen vierten Teil 25, welcher rechts unten an der ersten Manschette 2 angeordnet ist, strömt. Analog hierzu verläuft das Fluid in dem jeweiligen ersten, zweiten, dritten und vierten Teil 22', 23', 24', 25' in der zweiten Manschette 3. Durch diesen Strömungsverlauf wird eine optimierte wärmetherapeutische Versorgung der Brüste 31, 32 der Patientin 30 gewährleistet.

Mittels einer Übertragungseinrichtung 10 kann das Fluid im Bereich der Außenkante 19 des vierten Teils 25 von der ersten Manschette 2 zum ersten Teil 22' der zweiten Manschette 3 übertragen werden, um eine kontinuierliche Strömung des Fluids zwischen den beiden Manschetten 2, 3 zu gewährleisten. Nachdem das Fluid von dem ersten, zu dem zweiten, dritten und schließlich zum vierten Teil 22', 23', 24', 25' der zweiten Manschette 3 geströmt ist, kann dieses im Bereich der Außenkante 19 im vierten Teil 25' der zweiten Manschette 3 über eine zweite Zuleitung 9 abgeführt werden. Alternativ kann die Strömungsrichtung auch von der zweiten Manschette zur ersten Manschette erfolgen, wobei der eben geschilderte Strömungsverlauf des Fluids in den beiden Wärmeübertragungskörpern der beiden Manschetten 2, 3 umgekehrt wird. Ferner entsprechen sich die erste und zweite Zuleitung 8, 9 in der Anordnung an der ersten und zweiten Manschette 2, 3, sodass wenigstens im Wesentlichen eine gleiche Gewichtsverteilung vorherrscht.

Darüber hinaus sind exemplarisch verschiedene Verstelleinrichtungen im Bereich der Außenkante 19 bzw. der jeweiligen ersten und zweiten Einschnittkanten 20, 21 angeordnet. Zwischen dem ersten und dem zweiten Teil 22, 23 der ersten Manschette 2 ist hierbei eine Verstelleinrichtung als Klettverschluss 14, 14' ausgebildet, sodass der erste Teil 22 und der zweite Teil 23 wenigstens teilweise überlagert werden können, um die erste Manschette 2 an die Form der ersten Brust 31 der Patientin 30 anzupassen. Zwischen dem zweiten und dritten Teil 23, 24 der ersten Manschette 2 ist im Bereich des Einschnitts 18 am dritten Teil 24 eine weitere Verstelleinrichtung in der Form einer Schlaufe 15 ausgebildet, welche mit voneinander beabstandeten Knöpfen 16, 16', 16", 16", welche an dem zweiten Teil 22 angeordnet sind, verbindbar ist. Somit kann wiederum die erste Manschette 2 an die Form der ersten Brust 31 der Patientin 30 angepasst werden. Zwischen dem zweiten Teil 23' und dem dritten Teil 24' der zweiten Manschette 3 ist eine zusätzliche Verstelleinrichtung als Lochraster 13, 13', 13", 13‴ ausgebildet, womit die zweite Manschette 3 an die Form der zweiten Brust 32 der Patientin 30 angepasst werden kann. Ferner kann die Verstelleinrichtung auch als Knopf 16 oder auch Druckknopf ausgebildet sein. Insbesondere befindet sich zwischen jedem Einschnitt der beiden Manschetten 2, 3 eine Verstelleinrichtung, um eine optimierte Befestigung und Anpassung an die Brustform der Patientin 30 zu gewährleisten.

### BEZUGSZEICHENLISTE

- 1: Behandlungsvorrichtung
- 2: erste Manschette
- 3: zweite Manschette
- 4, 4', 4", 4‴: Verbindungseinrichtung
- 5: Befestigungseinrichtung
- 6, 6': erste Trageinrichtung
- 7, 7': zweite Trageinrichtung
- 8: erste Zuleitung
- 9: zweite Zuleitung
- 10: Übertragungseinrichtung
- 11: Fluidversorgungseinrichtung
- 12, 12': Strömungsraum
- 13, 13`, 13", 13‴: Lochraster
- 14, 14': Klettverschluss
- 15: Schlaufe
- 16, 16`, 16", 16‴: Knopf
- 17, 17': Kreismittelpunkt
- 18, 18', 18", 18‴: Einschnitt
- 19: Außenkante
- 20: erste Einschnittkante
- 21: zweite Einschnittkante
- 22, 22': erster Teil der Manschette
- 23, 23': zweiter Teil der Manschette
- 24, 24': dritter Teil der Manschette
- 25, 25': vierter Teil der Manschette
- 30: Patientin
- 31: erste Brust
- 32: zweite Brust
- 33: Brustbein
- 34: Brustkorb
- 35: Hals

## Patentansprüche

1. Behandlungsvorrichtung (1) zur Verwendung bei der gleichzeitigen wärmetherapeutischen Behandlung beider Brüste (31, 32) einer Patientin (30), insbesondere zur Verwendung bei der Behandlung nach operativen Eingriffen und/oder von Verletzungen und/oder von Entzündungen, mit
- einer ersten und einer zweiten Manschette (2, 3), welche jeweils wenigstens im Wesentlichen halbkugelförmig ausgebildet ist und mit welcher jeweils eine Brust (31; 32) weitgehend oder vollständig bedeckbar ist, wobei die erste Manschette (2) wenigstens eine erste flexible Materiallage und wenigstens eine zweite flexible Materiallage, welche einen ersten Wärmeübertragungskörper bilden, und die zweite Manschette (3) wenigstens eine dritte flexible Materiallage und wenigstens eine vierte flexible Materiallage, welche einen zweiten Wärmeübertragungskörper bilden, aufweist, wobei jeweils die erste und zweite flexible Materiallage der ersten Manschette (2) und die dritte und vierte flexible Materiallage der zweiten Manschette (3) einen oder mehrere von einem wärmeübertragenden Fluid durchströmbare Strömungsräume (12, 12') begrenzen und durch eine Vielzahl von Verbindungseinrichtungen (4, 4', 4", 4‴) miteinander verbunden sind;
- einer Befestigungseinrichtung (5), welche an einem ersten Ende mit der ersten Manschette (2) und an einem zweiten Ende mit der zweiten Manschette (3) verbindbar ist, und zwar im Bereich des Brustbeins (33) der Patientin (30), wenn die Behandlungsvorrichtung (1) während der Behandlung an der Patientin (30) angeordnet ist;
- einer ersten, flexiblen länglichen Trageinrichtung (6), welche an einem ersten Ende mit der ersten Manschette (2) und an einem zweiten Ende mit der zweiten Manschette (3) verbindbar ist, sodass durch die erste Trageinrichtung (6), wenn diese am Hals (35) der Patientin (30) angeordnet ist, die erste und zweite Manschette (2, 3) höhenverstellbar ist;
- einer zweiten, flexiblen länglichen Trageinrichtung (7), welche an einem ersten Ende mit der ersten Manschette (2) und an einem zweiten Ende mit der zweiten Manschette (3) verbindbar ist, sodass durch die zweite Trageinrichtung (7), wenn diese den Brustkorb (34) der Patientin (30) umschließt, die erste Manschette (2) an einer ersten Brust (31) und die zweite Manschette (3) an einer zweiten Brust (32) der Patientin (30) fixierbar ist;
- wenigstens einer ersten Zuleitung (8), um dem ersten und/oder zweiten Wärmeübertragungskörper ein wärmeübertragendes Fluid zuzuführen;
- wenigstens einer zweiten Zuleitung (9), um ein wärmeübertragendes Fluid von dem ersten und/oder zweiten Wärmeübertragungskörper abzuführen; und
- einer Übertragungseinrichtung (10), durch welche der erste Wärmeübertragungskörper mit dem zweiten Wärmeübertragungskörper verbindbar ist, sodass das wärmeübertragende Fluid von dem ersten in den zweiten Wärmeübertragungskörper und umgekehrt strömen kann,
**dadurch gekennzeichnet, dass** die Übertragungseinrichtung (10) im Bereich des Brustbeins (33) der Patientin (30) angeordnet ist, wenn die Behandlungsvorrichtung (1) während der Behandlung an der Patientin (30) angeordnet ist.

2. Behandlungsvorrichtung (1) nach Anspruch 1, wobei die Befestigungseinrichtung (5) und/oder die erste Trageinrichtung (6) und/oder die zweite Trageinrichtung (7) jeweils an einem ersten und/oder an einem zweiten Ende wenigstens ein Fixierungselement, insbesondere wenigstens einen Knopf (16) und/oder wenigstens einen Druckknopf und/oder wenigstens ein Lochraster (13) und/oder wenigstens einen Klettverschluss (14) und/oder wenigstens eine Schlaufe (15), aufweist, womit die Befestigungseinrichtung (5) und/oder die erste Trageinrichtung (6) und/oder die zweite Trageinrichtung (7) mit der ersten Manschette (2) und/oder der zweiten Manschette (3) verbindbar ist.

3. Behandlungsvorrichtung (1) nach Anspruch 2, wobei die Befestigungseinrichtung (5) und/oder die erste Trageinrichtung (6) und/oder die zweite Trageinrichtung (7) jeweils an einem ersten und/oder an einem zweiten Ende durch das wenigstens eine Fixierungselement längenverstellbar ist.

4. Behandlungsvorrichtung (1) nach wenigstens einem der vorhergehenden Ansprüche, wobei die erste und die zweite Manschette (2, 3) jeweils an wenigstens einer Stelle der Außenkante (19) einen Einschnitt (18) aufweisen, welcher in der Aufsicht der ersten oder der zweiten Manschette (2, 3 ) jeweils wenigstens im Wesentlichen bis zum Kreismittelpunkt (17, 17') der ersten und der zweiten Manschette (2, 3) ausgebildet ist und die erste und zweite Manschette (2, 3) an dem Einschnitt (18) jeweils eine erste und zweite Einschnittkante (20, 21) aufweist.

5. Behandlungsvorrichtung (1) nach wenigstens einem der vorhergehenden Ansprüche, wobei die erste und die zweite Manschette (2, 3) jeweils an wenigstens zwei, vorzugsweise drei, weiter vorzugsweise vier, voneinander beabstandeten Stellen der Außenkante (19) einen Einschnitt (18, 18', 18", 18‴) aufweisen, wobei jeweils die wenigstens zwei Einschnitte (18, 18') der ersten und der zweiten Manschette (2, 3) in der Aufsicht der ersten oder der zweiten Manschette (2, 3) jeweils wenigstens im Wesentlichen bis zum Kreismittelpunkt (17, 17') der ersten und der zweiten Manschette (2, 3) ausgebildet sind und die erste und die zweite Manschette (2, 3) jeweils in wenigstens einen ersten und einen zweiten Teil (22, 22', 23, 23') separieren, wobei die jeweiligen wenigstens ersten und wenigstens zweiten Teile (22, 22', 23, 23') der ersten und zweiten Manschette (2, 3) lediglich im Bereich des Kreismittelpunkts (17, 17') stoffschlüssig miteinander verbunden sind, und die erste und zweite Manschette (2, 3) an den Einschnitten (18, 18') jeweils eine erste und zweite Einschnittkante (20, 21) aufweisen.

6. Behandlungsvorrichtung (1) nach Anspruch 4 oder 5, wobei die erste und die zweite Einschnittkante (20, 21) wenigstens jeweils eines Einschnitts (18, 18') der ersten und der zweiten Manschette (2, 3) jeweils mittels wenigstens einer Verstelleinrichtung, insbesondere wenigstens einem Knopf (16) und/oder wenigstens einem Druckknopf und/oder wenigstens einem Lochraster (13) und/oder wenigstens einem Klettverschluss (14) und/oder wenigstens einer Schlaufe (15), verbindbar sind, sodass die erste Manschette (2) an die erste Brust (31) und die zweite Manschette (3) an die zweite Brust (32) der Patientin (30) anpassbar ist.

7. Behandlungsvorrichtung (1) nach Anspruch 5 oder 6, wobei die Strömungsräume (12, 12') wenigstens im Wesentlichen mäanderförmig, insbesondere jeweils von dem wenigstens ersten Teil (22, 22') der ersten oder der zweiten Manschette (2, 3) zu dem wenigstens zweiten Teil (23, 23') der ersten oder der zweiten Manschette (2, 3), verlaufen.

8. Behandlungsvorrichtung (1) nach wenigstens einem der vorhergehenden Ansprüche, wobei die erste Zuleitung (8) und die zweite Zuleitung (9) im Wesentlichen jeweils an einem Bereich des ersten und des zweiten Wärmeübertragungskörpers angeschlossen sind, welche sich in Bezug auf den Aufbau der ersten und zweiten Manschette (2, 3), insbesondere spiegelsymmetrisch, entsprechen.

9. Behandlungsvorrichtung (1) nach wenigstens einem der vorhergehenden Ansprüche, wobei die Befestigungseinrichtung (5) und/oder die erste Trageinrichtung (6) und/oder die zweite Trageinrichtung (7) ein Kunstfasermaterial aufweisen.

10. Behandlungsvorrichtung (1) nach wenigstens einem der vorhergehenden Ansprüche, wobei die Befestigungseinrichtung (5) und/oder die erste Trageinrichtung (6) und/oder die zweite Trageinrichtung (7) einen Kunststoff aufweisen.

11. Behandlungsvorrichtung (1) nach wenigstens einem der vorhergehenden Ansprüche, wobei die Vielzahl von Verbindungseinrichtungen (4", 4‴) zumindest teilweise rasterförmig, vorzugsweise an den Eckpunkten von gedachten Rechtecken, weiter vorzugsweise an den Eckpunkten von gedachten Quadraten, angeordnet ist.

12. Wärmebehandlungssystem zur Verwendung bei der wärmetherapeutischen Behandlung beider Brüste (31, 32) einer Patientin (30) mit einer Behandlungsvorrichtung (1) nach wenigstens einem der Ansprüche 1 bis 11 und mit einer Fluidversorgungseinrichtung (11), die mit der Behandlungsvorrichtung (1) fluidleitend verbindbar ist.

13. Wärmebehandlungssystem nach Anspruch 12, wobei die Fluidversorgungseinrichtung (11) mit wenigstens einer ersten Zuleitung (8) der Behandlungsvorrichtung (1) und wenigstens einer zweiten Zuleitung (9) der Behandlungsvorrichtung (1) fluidleitend verbindbar ist und dazu eingerichtet ist, ein wärmeübertragendes Fluid auf eine für die Wärmebehandlung geeignete Temperatur, bevorzugt eine Temperatur zwischen +5 °C und +35 °C, weiter bevorzugt zwischen +15 °C und +22 °C, besonders bevorzugt zwischen +17 °C und +19 °C und ganz besonders bevorzugt um ca. +18 °C, zu temperieren, das derart temperierte wärmeübertragende Fluid einem ersten Wärmeübertragungskörper oder einem zweiten Wärmeübertragungskörper der Behandlungsvorrichtung (1) über die erste Zuleitung (8) zuzuführen und das wärmeübertragende Fluid von dem ersten Wärmeübertragungskörper oder dem zweiten Wärmeübertragungskörper über die zweite Zuleitung (9) abzuführen.

14. Verwendung einer Behandlungsvorrichtung (1) nach einem der Ansprüche 1 bis 11 und/oder eines Wärmebehandlungssystems nach Anspruch 12 zur kosmetischen Behandlung der Haut von Brüsten (31, 32) einer Patientin (30), insbesondere zur thermischen Beeinflussung der Kapillarzeichnung auf der Haut.

## Claims

1. Treatment device (1) for use in the simultaneous heat-therapeutic treatment of both breasts (31, 32) of a female patient (30), in particular for use in the treatment after surgical interventions and/or of injuries and/or of inflammations, with
- a first and a second sleeve (2, 3), each of which is at least substantially hemispherically shaped and with each of which a breast (31; 32) can be largely or completely covered, the first sleeve (2) having at least one first flexible material layer and at least one second flexible material layer, which form a first heat transfer body, and the second sleeve (3) having at least one third flexible material layer and at least one fourth flexible material layer, which form a second heat transfer body, the first and second flexible material layers of the first sleeve (2) and the third and fourth flexible material layers of the second sleeve (3) respectively delimiting one or more flow spaces (12, 12') through which a heat-transmitting fluid can flow and being connected to one another by a multiplicity of connecting devices (4, 4', 4", 4‴);
- an attachment device (5) connectable at a first end to the first sleeve (2) and at a second end to the second sleeve (3), in the region of the sternum (33) of the female patient (30) when the treatment device (1) is placed on the female patient (30) during treatment;
- a first, flexible elongate support device (6) connectable at a first end to the first sleeve (2) and at a second end to the second sleeve (3) such that the first support device (6), when positioned on the neck (35) of the female patient (30), allows the first and second sleeves (2, 3) to be adjusted in height;
- a second, flexible elongate support device (7) which can be connected at a first end to the first sleeve (2) and at a second end to the second sleeve (3), so that the second support device (7), when it encloses the thorax (34) of the female patient (30), can fix the first sleeve (2) to a first breast (31) and the second sleeve (3) to a second breast (32) of the female patient (30);
- at least one first supply line (8) for supplying a heat-transferring fluid to the first and/or second heat transfer body;
- at least one second supply line (9) for discharging a heat transfer fluid from the first and/or second heat transfer body; and
- a transfer device (10) by which the first heat transfer body is connectable to the second heat transfer body so that the heat transfer fluid can flow from the first into the second heat transfer body and vice versa,
**characterized in that** the transfer device (10) is arranged in the region of the sternum (33) of the female patient (30) when the treatment device (1) is arranged on the female patient (30) during treatment.

2. Treatment device (1) according to claim 1, wherein the attachment device (5) and/or the first support device (6) and/or the second support device (7) each has at least one fixing element at a first end and/or at a second end, in particular at least one button (16) and/or at least one push button and/or at least one perforated grid (13) and/or at least one Velcro fastener (14) and/or at least one loop (15), by means of which the attachment device (5) and/or the first support device (6) and/or the second support device (7) can be connected to the first sleeve (2) and/or the second sleeve (3).

3. Treatment device (1) according to claim 2, wherein the attachment device (5) and/or the first support device (6) and/or the second support device (7) can each be adjusted in length at a first end and/or at a second end by the at least one fixing element.

4. Treatment device (1) according to at least one of the preceding claims, wherein the first and the second sleeve (2, 3) each have, at at least one location of the outer edge (19), an incision (18) which, in the top view of the first or the second sleeve (2, 3), is formed in each case at least substantially as far as the circle center (17, 17') of the first and the second sleeve (2, 3), and the first and the second sleeve (2, 3) each have, at the incision (18), a first and a second incision edge (20, 21).

5. Treatment device (1) according to at least one of the preceding claims, wherein the first and the second sleeve (2, 3) each have an incision (18, 18', 18", 18‴) at at least two, preferably three, further preferably four, spaced-apart points of the outer edge (19), wherein the at least two incisions (18, 18') of the first and of the second sleeve (2, 3) in the top view of the first or of the second sleeve (2, 3) are each formed at least substantially as far as the circle center (17, 17') of the first and the second sleeve (2, 3) and separate the first and the second sleeve (2, 3) respectively into at least a first and a second part (22, 22', 23, 23'), wherein the respective at least first and at least second parts (22, 22', 23, 23') of the first and second sleeves (2, 3) are connected to one another in a materially bonded manner only in the region of the circle center (17, 17'), and the first and second sleeves (2, 3) each have a first and second incision edge (20, 21) at the incisions (18, 18').

6. Treatment device (1) according to claim 4 or 5, wherein the first and the second incision edge (20, 21) of at least one incision (18, 18') of the first and the second sleeve (2, 3) are each connectable by means of at least one adjusting device, in particular at least one button (16) and/or at least one press button and/or at least one perforated grid (13) and/or at least one Velcro fastener (14) and/or at least one loop (15), so that the first sleeve (2) can be adapted to the first breast (31) and the second sleeve (3) can be adapted to the second breast (32) of the female patient (30).

7. Treatment device (1) according to claim 5 or 6, wherein the flow spaces (12, 12') extend at least substantially in a meandering manner, in particular in each case from the at least first part (22, 22') of the first or the second sleeve (2, 3) to the at least second part (23, 23') of the first or the second sleeve (2, 3).

8. Treatment device (1) according to at least one of the preceding claims, wherein the first supply line (8) and the second supply line (9) are substantially each connected to a region of the first and the second heat transfer body, which correspond to each other with respect to the structure of the first and second sleeve (2, 3), in particular mirror-symmetrically.

9. Treatment device (1) according to at least one of the preceding claims, wherein the attachment device (5) and/or the first support device (6) and/or the second support device (7) comprise a synthetic fiber material.

10. Treatment device (1) according to at least one of the preceding claims, wherein the attachment device (5) and/or the first support device (6) and/or the second support device (7) comprise a plastic.

11. Treatment device (1) according to at least one of the preceding claims, wherein the plurality of connecting devices (4", 4‴) is arranged at least partially in a grid-like manner, preferably at the corner points of imaginary rectangles, further preferably at the corner points of imaginary squares.

12. Heat treatment system for use in the heat therapeutic treatment of both breasts (31, 32) of a female patient (30), comprising a treatment device (1) according to at least one of claims 1 to 11 and a fluid supply device (11) connectable to the treatment device (1) in a fluid-conducting manner.

13. Heat treatment system according to claim 12, wherein the fluid supply device (11) is connectable in a fluid-conducting manner to at least one first supply line (8) of the treatment device (1) and at least one second supply line (9) of the treatment device (1) and is set up to temper a heat-transmitting fluid to a temperature suitable for the heat treatment, preferably a temperature between +5 °C and +35 °C, further preferably between +15 °C and +22 °C, particularly preferably between +17 °C and +19 °C and very particularly preferably of approximately +18 °C, to supply the heat-transmitting fluid thus tempered to a first heat transfer body or a second heat transfer body of the treatment device (1) via the first supply line (8) and to discharge the heat-transmitting fluid from the first heat transfer body or the second heat transfer body via the second supply line (9).

14. Use of a treatment device (1) according to any one of claims 1 to 11 and/or of a heat treatment system according to claim 12 for cosmetic treatment of the skin of breasts (31, 32) of a female patient (30), in particular to thermally influence the capillary drawing on the skin.

## Revendications

1. Dispositif de traitement (1) destiné à être utilisé dans le traitement par thermothérapie simultané des deux seins (31, 32) d'une patiente (30), en particulier destiné à être utilisé le traitement après des interventions chirurgicales et/ou des blessures et/ou des inflammations, avec
- un premier et un second manchon (2, 3), qui sont réalisés respectivement au moins sensiblement en forme de demi-sphère et avec lesquels un sein (31 ; 32) peut être recouvert dans une large mesure ou en totalité, dans lequel le premier manchon (2) présente au moins une première couche de matériau flexible et au moins une deuxième couche de matériau flexible, lesquelles forment un premier corps de transfert de chaleur, et le second manchon (3) présente au moins une troisième couche de matériau flexible et au moins une quatrième couche de matériau flexible, lesquelles forment un deuxième corps de transfert de chaleur, dans lequel la première et la deuxième couche de matériau flexible du premier manchon (2) et la troisième et la quatrième couche de matériau flexible du second manchon (3) délimitent respectivement une ou plusieurs chambres d'écoulement (12, 12') pouvant être traversées par un fluide caloporteur et sont reliées entre elles par une pluralité de systèmes de liaison (4, 4', 4", 4"") ;
- un système de fixation (5), qui peut être relié sur une première extrémité au premier manchon (2) et sur une seconde extrémité au second manchon (3), à savoir dans la région du sternum (33) de la patiente (30), lorsque le dispositif de traitement (1) est disposé sur la patiente (30) pendant le traitement ;
- un premier système porteur allongé flexible (6), lequel peut être relié sur une première extrémité au premier manchon (2) et sur une seconde extrémité au second manchon (3) si bien que le premier système porteur (6), lorsque celui-ci est disposé au niveau du cou (35) de la patiente (30), permet d'ajuster en hauteur le premier et le second manchon (2, 3) ;
- un deuxième système porteur allongé flexible (7), qui peut être relié sur une première extrémité au premier manchon (2) et sur une seconde extrémité au second manchon (3), si bien que le deuxième système porteur (7), lorsque celui-ci entoure le thorax (34) de la patiente (30), permet de bloquer le premier manchon (2) sur un premier sein (31) et le second manchon (3) sur un second sein (32) de la patiente (30) ;
- au moins une première conduite d'amenée (8) pour amener au premier et/ou au deuxième corps de transfert de chaleur un fluide caloporteur ;
- au moins une deuxième conduite d'amenée (9) pour évacuer un fluide caloporteur du premier et/ou du deuxième corps de transfert de chaleur ; et
- un système de transfert (10), par lequel le premier corps de transfert de chaleur peut être relié au deuxième corps de transfert de chaleur si bien que le fluide caloporteur peut s'écouler du premier dans le deuxième corps de transfert de chaleur, et vice versa,
**caractérisé en ce que** le système de transfert (10) est disposé dans la région du sternum (33) de la patiente (30) lorsque le dispositif de traitement (1) est disposé sur la patiente (30) pendant le traitement.

2. Dispositif de traitement (1) selon la revendication 1, dans lequel le système de fixation (5) et/ou le premier système porteur (6) et/ou le deuxième système porteur (7) présentent respectivement sur une première et/ou sur une seconde extrémité au moins un élément de blocage, en particulier au moins un bouton (16) et/ou au moins un bouton-poussoir et/ou au moins une trame perforée (13) et/ou au moins une fermeture velcro (14) et/ou au moins une boucle (15), de sorte que le système de fixation (5) et/ou le premier système porteur (6) et/ou le deuxième système porteur (7) peuvent être reliés au premier manchon (2) et/ou au second manchon (3).

3. Dispositif de traitement (1) selon la revendication 2, dans lequel le système de fixation (5) et/ou le premier système porteur (6) et/ou le deuxième système porteur (7) peuvent être ajustés en longueur respectivement sur une première et/ou sur une seconde extrémité par l'au moins un élément de blocage.

4. Dispositif de traitement (1) selon au moins l'une quelconque des revendications précédentes, dans lequel le premier et le second manchon (2, 3) présentent respectivement sur au moins un emplacement du bord extérieur (19) une entaille (18), qui est réalisée sous la surveillance du premier ou du second manchon (2, 3) respectivement au moins sensiblement jusqu'au centre du cercle (17, 17') du premier et du second manchon (2, 3) et le premier et le second manchon (2, 3) présentent respectivement un premier et un second bord d'entaille (20, 21) sur l'entaille (18).

5. Dispositif de traitement (1) selon au moins l'une quelconque des revendications précédentes, dans lequel le premier et le second manchon (2, 3) présentent respectivement sur au moins deux, de préférence trois, de préférence par ailleurs quatre, emplacements du bord extérieur (19) espacés les uns des autres une entaille (18, 18', 18", 18""), dans lequel respectivement les au moins deux entailles (18, 18') du premier et du second manchon (2, 3) sont réalisées sous a surveillance du premier ou du second manchon (2, 3) respectivement au moins sensiblement jusqu'au centre du cercle (17, 17') du premier et du second manchon (2, 3) et séparent le premier et le second manchon (2, 3) respectivement en au moins une première et une deuxième partie (22, 22', 23, 23'), dans lequel l'au moins une première et l'au moins une deuxième partie (22, 22', 23, 23') respective du premier et du second manchon (2, 3) ne sont reliées l'une à l'autre par liaison de matière que dans la zone du centre du cercle (17, 17') et le premier et le second manchon (2, 3) présentent respectivement un premier et un second bord d'entaille (20, 21) sur les entailles (18, 18').

6. Dispositif de traitement (1) selon la revendication 4 ou 5, dans lequel le premier et le second bord d'entaille (20, 21) d'au moins respectivement une entaille (18, 18') du premier et du second manchon (2, 3) peuvent être reliés respectivement au moyen d'au moins un système d'ajustement, en particulier d'au moins un bouton (16) et/ou d'au moins un bouton-poussoir et/ou d'au moins une trame perforée (13) et/ou d'au moins une fermeture velcro (14) et/ou d'au moins une boucle (15), si bien que le premier manchon (2) peut être adapté au premier sein (31) et le second manchon (3) au second sein (32) de la patiente (30).

7. Dispositif de traitement (1) selon la revendication 5 ou 6, dans lequel les chambres d'écoulement (12, 12') s'étendent au moins sensiblement sous forme de méandres, en particulier respectivement depuis l'au moins une première partie (22, 22') du premier et du second manchon (2, 3) à l'au moins une deuxième partie (23, 23') du premier ou du second manchon (2, 3).

8. Dispositif de traitement (1) selon au moins l'une quelconque des revendications précédentes, dans lequel la première conduite d'amenée (8) et la deuxième conduite d'amenée (9) sont raccordées sensiblement respectivement à une zone du premier et du deuxième corps de transfert de chaleur, lesquels correspondent, en ce qui concerne la structure, au premier et au second manchon (2, 3), en particulier de manière symétrique en miroir.

9. Dispositif de traitement (1) selon au moins l'une quelconque des revendications précédentes, dans lequel le système de fixation (5) et/ou le premier système porteur (6) et/ou le deuxième système porteur (7) présentent un matériau en fibres synthétiques.

10. Dispositif de traitement (1) selon au moins l'une quelconque des revendications précédentes, dans lequel le système de fixation (5) et/ou le premier système porteur (6) et/ou le deuxième système porteur (7) présentant une matière plastique.

11. Dispositif de traitement (1) selon au moins l'une quelconque des revendications précédentes, dans lequel la pluralité de système de liaison (4", 4"") est disposée au moins en partie sous la forme d'une trame, de préférence aux coins de rectangles imaginaires, de préférence aux coins de carrés imaginaires.

12. Système de traitement thermique destiné à être utilisé dans le traitement par thermothérapie des deux seins (31, 32) d'une patiente (30) avec un dispositif de traitement (1) selon au moins l'une quelconque des revendications 1 à 11 et avec un système d'alimentation en fluide (11), qui peut être relié en liaison fluidique au dispositif de traitement (1).

13. Système de traitement thermique selon la revendication 12, dans lequel le système d'alimentation en fluide (11) peut être relié en communication fluidique à au moins une première conduite d'amenée (8) du dispositif de traitement (1) et à au moins une deuxième conduite d'amenée (9) du dispositif de traitement (1) et est mis au point pour thermoréguler un fluide caloporteur à une température adaptée au traitement thermique, de préférence à une température comprise entre +5 °C et +35 °C, de préférence entre +15 °C et +22 °C, de manière particulièrement préférée entre +17 °C et +19 °C, et de manière très particulièrement préférée d'environ +18 °C, pour amener le fluide caloporteur ainsi thermorégulé à un premier corps de transfert thermique ou à un deuxième corps de transfert thermique du dispositif de traitement (1) par l'intermédiaire de la première conduite d'amenée (8) et d'évacuer le fluide caloporteur du premier corps de transfert thermique ou du deuxième corps de transfert thermique par l'intermédiaire de la deuxième conduite d'amenée (9).

14. Utilisation d'un dispositif de traitement (1) selon l'une quelconque des revendications 1 à 11 et/ou d'un système de traitement thermique selon la revendication 12 pour le traitement cosmétique de la peau des seins (31, 32) d'une patiente (30) pour influencer thermiquement le dessin capillaire sur la peau.
